# EUROPEAN PATENT SPECIFICATION

(11) **EP 2 018 823 B1**
(45) Date of publication and mention of the grant of the patent: **20.10.2010**
(21) Application number: 08011431.7
(22) Date of filing: 24.06.2008
(51) Int. Cl.: A61L 2/18, A61B 1/12

(54) **Endoscope cleaner**
Endoskopreiniger
Nettoyant d'endoscope

(30) Priority: 25.06.2007 JP 2007166440
(43) Date of publication of application: 28.01.2009
(73) Proprietor: FUJIFILM Corporation, Minato-ku Tokyo (JP)
(72) Inventor: Miyako, Kuniaki, Ashigarakami-gun Kanagawa (JP)
(74) Representative: Klunker . Schmitt-Nilson . Hirsch

(56) References cited:
- WO-A-01/56615
- US-A1- 2004 091 389
- US-B1- 6 485 684

## Description

### BACKGROUND OF THE INVENTION

The present invention relates to an endoscope cleaner according to the preamble of claim 1. In particular, the present invention relates to an endoscope cleaner by means of which a plurality of endoscopes can be individually cleaned and disinfected in an automatic fashion.

As is well known, the endoscope is inserted into cavities in the human body or other living bodies for use in various applications including the diagnosis and treatment of organs, and the sampling of specimens.

Endoscopes are used on more than one patient and repeatedly. Hence, used endoscopes require thorough hygienic management and to ensure complete prevention of troubles such as endoscope-mediated bacterial infection, they must be meticulously cleaned and disinfected after every use.

The endoscope cleaner has already been commercialized as an apparatus for automatic cleaning and disinfection of endoscopes in a way that is not only accurate but also safe to the operator.

If the frequency of an endoscope's use is high, a plurality of endoscopes are used and then cleaned and disinfected sequentially. However, cleaning and disinfecting endoscopes involve time-consuming procedures and particularly in the case of disinfection, it is strictly required to immerse endoscopes in a liquid disinfectant for a certain period of time. So, depending on the frequency of an endoscope's use, it is necessary to use more than one unit of endoscope cleaner. In addition, an apparatus equipped with two cleaning stations having the same configuration is known as a system capable of cleaning two different endoscopes simultaneously or consecutively (see JP 2006-334405 A).

There has also been proposed an apparatus for cleaning and disinfecting biopsy forceps and other treatment tools for use with an endoscope; this cleaning and disinfecting apparatus has a plurality of individual cleaning and disinfecting vessels that are independently partitioned in the main body and these vessels are independently or simultaneously operated for cleaning or disinfecting the treatment tools (see JP 2000-217781 A).

In accordance with the preamble of claim 1, US 2004/091389 discloses an endoscope cleaner, with two or more reprocessing bags, a reaction chamber providing disinfectant to the bags and a collection and recirculation system for recirculating 2-5 liters of disinfectant fluid.

### SUMMARY OF THE INVENTION

However, plural units of endoscope cleaner having the same configuration require a very large area for installation. In addition, a disinfectant and other liquid chemicals must be provided and managed for each unit. The same problems occur to the apparatus described in JP 2006-334405 A which is equipped with two cleaning stations having the same configuration.

The apparatus described in JP 2000-217781 A supplies a disinfectant from a single disinfectant tank for use in a plurality of cleaning and disinfecting vessels, so the disinfectant can be managed in an integrated fashion. However, this apparatus discards the disinfectant once it is used, so it is unable to cope with the need to use the disinfectant in more than one step of disinfection.

An object, therefore, of the present invention is to solve the above-mentioned problems of the prior art by providing an endoscope cleaner that can clean and disinfect a plurality of endoscopes individually and which is also capable of integrated management of a disinfectant that is to be used in more than one step of disinfection in a plurality of cleaning vessels.

In order to achieve the object, the present invention provides an endoscope cleaner comprising the features of claim 1.

Preferably, the first number of the at least one disinfectant tank is unity, or the second number of the plurality of cleaning vessels is two.

Preferably, the control unit controls the steps of the cleaning and disinfecting treatment in two or more cleaning vessels that share a specific disinfectant tank of the at least one disinfectant tank such that the two or more cleaning vessels will not be supplied with the disinfectant from the specific disinfectant tank simultaneously.

Preferably, before a cleaning vessel in the two or more cleaning vessels that share the specific disinfectant tank starts a disinfecting step, the control unit checks if a remainder of the two or more cleaning vessels is being supplied with the disinfectant and, if the disinfectant is being supplied, causes the cleaning vessel not to start the disinfecting step until after the disinfectant is completely supplied and, if the disinfectant is not being supplied, causes the cleaning vessel to start the disinfecting step.

Preferably, the control unit checks if the two or more cleaning vessels that share the specific disinfectant tank have been set for a preferential cleaning mode and controls the steps of the cleaning and disinfecting treatment in the two or more cleaning vessels such that supply of the disinfectant into other cleaning vessel than a specific cleaning vessel that has been set for the preferential cleaning mode will be started after the disinfectant is completely supplied into the specific cleaning vessel that has been set for the preferential cleaning mode.

Preferably, the control unit controls the steps of the cleaning and disinfecting treatment in two or more cleaning vessels that share a specific disinfectant tank of the at least one disinfectant tank such that the two or more cleaning vessels will not execute the disinfecting step simultaneously.

Preferably, before a cleaning vessel in the two or more cleaning vessels that share the specific disinfectant tank starts a disinfecting step, the control unit checks if a remainder of the two or more cleaning vessels is in the disinfecting step and, if it is in the disinfecting step, causes the cleaning vessel not to start the disinfecting step until after the disinfecting step in the remainder of the two or more cleaning vessels is completed and, if it is not in the disinfecting step, causes the cleaning vessel to start the disinfecting step.

Preferably, the control unit checks if the two or more cleaning vessels that share the specific disinfectant tank have been set for a preferential cleaning mode and controls the steps of the cleaning and disinfecting treatment in the two or more cleaning vessels such that the disinfecting step in other cleaning vessel than a specific cleaning vessel that has been set for the preferential cleaning mode will be started after the disinfecting step in the specific cleaning vessel that has been set for the preferential cleaning mode is completed.

According to the present invention, a plurality of endoscopes can be cleaned and disinfected individually and, what is more, a disinfectant that is to be used in more than one step of disinfection in a plurality of cleaning vessels can be managed in an integrated fashion.

### BRIEF DESCRIPTION OF THE INVENTION

FIG. 1 is a block diagram showing a schematic configuration of a first embodiment of the endoscope cleaner of the present invention.
FIG. 2 is a perspective view of an endoscope.
FIG. 3 is a block diagram showing an outline of the pipelines in the endoscope cleaner shown in FIG. 1.
FIG. 4 is a block diagram showing in concept a schematic configuration of the control unit.
FIG. 5 is a block diagram showing the essential configuration of the first embodiment of the present invention.
FIG. 6 is a block diagram showing the essential configuration of a modification of the first embodiment of the present invention.
FIG. 7 is a flow sheet showing an example of a cleaning and disinfecting treatment according to the first embodiment of the present invention.
FIG. 8 is a flow sheet showing another example of the cleaning and disinfecting treatment according to the first embodiment of the present invention.
FIG. 9 is a block diagram showing the essential configuration of a second embodiment of the present invention.
FIG. 10 is a block diagram showing the essential configuration of a modification of the second embodiment of the present invention.
FIG. 11 is a flow sheet showing an example of a cleaning and disinfecting treatment according to the second embodiment of the present invention:
FIG. 12 is a flow sheet showing another example of the cleaning and disinfecting treatment according to the second embodiment of the present invention.

### DETAILED DESCRIPTION OF THE INVENTION

The endoscope cleaner according to the present invention is described below in detail with reference to the preferred embodiments depicted in the accompanying drawings.

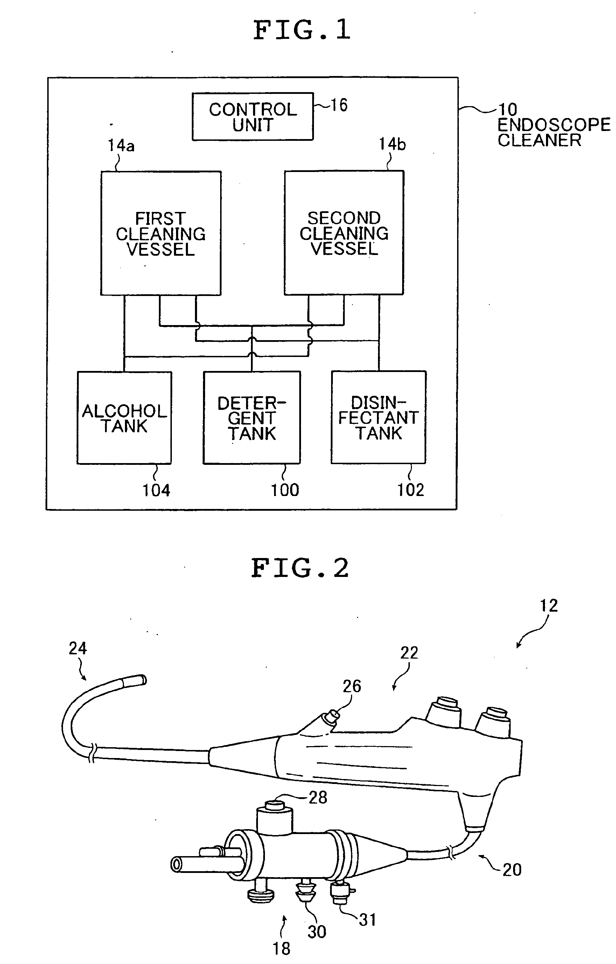

FIG. 1 is a block diagram showing a schematic configuration of a first embodiment of the endoscope cleaner of the present invention. The endoscope cleaner which is generally indicated by 10 in FIG. 1 (and which is hereinafter referred to as cleaner 10) is such an apparatus that two units of an endoscope which is generally indicated by 12 in FIG. 2 can be individually cleaned and disinfected. As shown, cleaner 10 comprises a first cleaning vessel 14a, a second cleaning vessel 14b, a control unit 16, a detergent tank 100, a disinfectant tank 102, and an alcohol tank 104.

Endoscope 12 is of a conventionally known type and, as shown in FIG. 2, it comprises a connector section 18 to be connected to a light source, a universal cord section 20 connected to the connector section 18, a manipulating section 22 that is connected to the universal cord section 20 for adjusting the viewing angle and effecting various operations such as suction and air insufflation or water purge, and an insertion section 24 that is connected to the manipulating section 22 and is to be inserted into body cavities of a patient.

A light guide is accommodated within the endoscope 12 to extend from the end of the connector section 18 to the distal end of the insertion section 24; an air insufflation or water purge tube that forms an air insufflation or water purge channel extends from an air insufflation or water purge channel opening 28 in the connector section 18 to the distal end of the insertion section 24; and a suction tube that forms a suction channel extends from a suction channel opening 30 in the connector section 18 to the distal end of the insertion section 24. In addition, a forceps channel is provided to extend from a forceps channel opening 26 in the manipulating section 22 to the distal end of the insertion section 24. In some models, a forceps raising channel is provided to extend from the forceps channel opening 26 in the manipulating section 22 to the distal end of the insertion section 24. In addition, a pressurizing opening 31 for water leak detection is provided at the connector section 18.

The procedure of cleaning and disinfecting the endoscope 12 involves thorough cleaning and disinfection of not only its exterior but also the interiors of the forceps channel, air insufflation or water purge channel, suction channel, and forceps raising channel. During this procedure, the electronic components for imaging need be fully protected from the cleaning water and the processing fluid.

Referring to cleaner 10 in FIG. 1, each of the first cleaning vessel 14a and the second cleaning vessel 14b is a vessel for cleaning and disinfecting one endoscope 12 and these vessels have the same configuration. The control unit 16 controls the steps-in the cleaning and disinfecting procedure to be followed in the first cleaning vessel 14a and the second cleaning vessel 14b.

The detergent tank 100 is a part where a detergent for cleaning the endoscope is held. The detergent is used in the first cleaning vessel 14a and the second cleaning vessel 14b after it is diluted with water by a predetermined number of folds. The diluted detergent (processing fluid) is used to clean the endoscope 12 and discharged as liquid waste after each cleaning step.

The disinfectant tank 102 is a part where the disinfectant is held. The disinfectant to be used in the cleaner 10 can withstand more than one cycle of disinfection. Therefore, the disinfectant supplied from the disinfectant tank 102 to the first cleaning vessel 14a or the second cleaning vessel 14b is recovered into the disinfectant tank 102 after disinfection. After it is used for a predetermined number of times, the disinfectant is discharged as liquid waste.

The alcohol tank 1034 is a part where an alcohol for alcohol flushing is held.

The cleaner 10 is equipped with one disinfectant tank 102 for the two cleaning vessels 14a and 14b, so these two cleaning vessels 14a and 14b share one and the same disinfectant tank 102. The cleaner 10 is also equipped with one unit each of detergent tank 100 and alcohol tank 104, which are shared by the two cleaning vessels 14a and 14b. In FIG. 1, the lines connecting each of the first cleaning vessel 14a and the second cleaning vessel 14b to the detergent tank 100, disinfectant tank 102 and the alcohol tank 104 indicate that these components are connected via pipes.

Note that the endoscope cleaner of the present invention may be equipped with three or more cleaning vessels, in which case one or more disinfectant tanks may be provided, with their number being less than that of the cleaning vessels. For example, if three cleaning vessels are used, they may share one disinfectant tank; alternatively, two disinfectant tanks may be provided such that one of them is shared by two of the three cleaning vessels. In short, the endoscope cleaner of the present invention is characterized in that at least two of the cleaning vessels share one disinfectant tank.

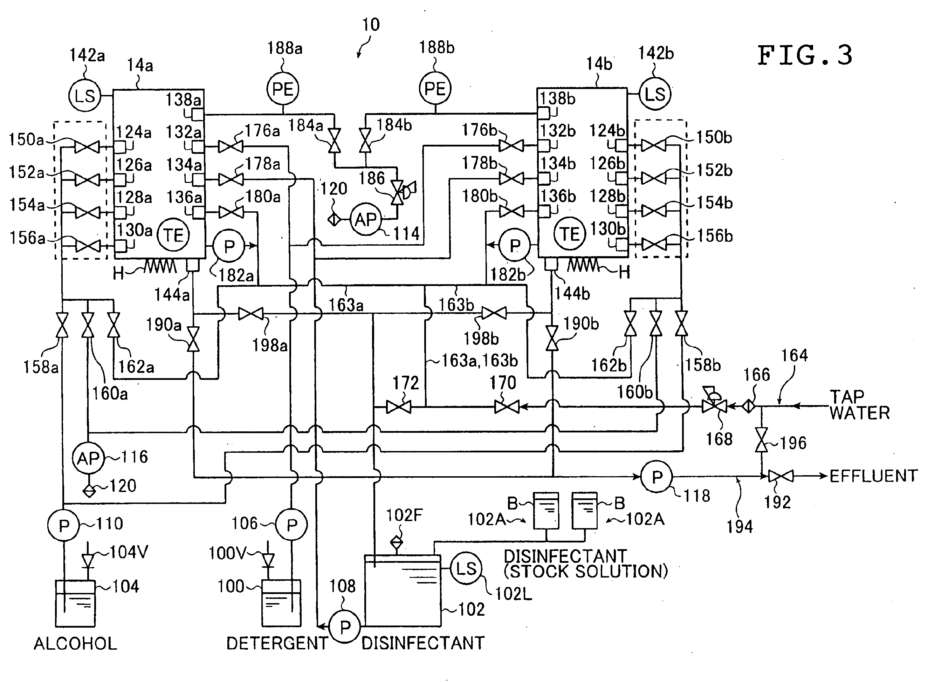

FIG. 3 is a block diagram showing an outline of the pipelines in the endoscope cleaner 10 shown in FIG. 1. As shown in FIG. 3, the cleaner 10 has only one unit each of a detergent pump 106, a disinfectant pump 108, and an alcohol pump 110; the detergent pump 106 supplies the detergent from the detergent tank 100 to the cleaning vessels 14a and 14b; the disinfectant pump 108 supplies the disinfectant from the disinfectant tank 102 to the cleaning vessels 14a and 14b; and the alcohol pump 110 supplies the alcohol from the alcohol tank 104 to the cleaning vessels 14a and 14b; each of the detergent pump 106, disinfectant pump 108, and the alcohol pump 110 is shared by the two cleaning vessels 14a and 14b.

These pumps may be of any various known types but it is of course preferred to use metering pumps. If the respective tanks are positioned below the cleaning vessels 14a and 14b, it is preferred to use self-priming metering pumps such as diaphragm pumps.

The cleaner 10 also has only one unit each of a first air pump 114, a second air pump 116, and a drain pump 118; the first air pump 114 is used to detect any water leak from the respective channels through the endoscope 12; the second air pump 116 is used to supply air into the respective channels through the endoscope 12; the drain pump 118 is used to drain the cleaning vessels 14a and 14b of water or the processing fluid; each of the first air pump 114, second air pump 116, and the drain pump 118 is shared by the two cleaning vessels 14a and 14b. Each of the first air pump 114 and the second air pump 116 is provided with an air filter 120 at the air intake.

In the illustrated case, the disinfectant tank 102 is provided with a level sensor 102L for measuring the volume of the disinfectant in the tank and an attachment 102A for mounting a bottle B that is filled with the disinfectant which is to be supplied into the disinfectant tank 102. In the illustrated exemplary case, the cleaner 10 is equipped with two attachments 102A, 102A. The disinfectant tank 102 is also equipped with a masking filter 102F that prevents the smell of the disinfectant from leaking to the outside. If desired, the disinfectant tank 102 may further have an air filter for preventing dust, miscellaneous germs and other foreign matter from mixing into the disinfectant tank 102.

The cleaner 10 may be so adapted that the disinfectant bottle B can be kept mounted until it is replaced by the next charge of disinfectant; in this case, the disinfectant bottle B acts as a lid for the attachment 102A, hence, for the disinfectant tank 102.

The detergent tank 100 is provided with a check valve 100V to prevent accidental draining of the detergent from the detergent tank 100, and the alcohol tank 104 is also provided with a check valve 104V to prevent accidental draining of the alcohol from the alcohol tank 104.

The first cleaning vessel 14a and the second cleaning vessel 14b have basically the same configuration and many parts of the piping systems are also the same; therefore, on the following pages, only the first cleaning vessel 14a will be described and as regards the second cleaning vessel 14b, the numerals or symbols for the corresponding constituents will be parenthesized and only those parts that have different configurations than the first cleaning vessel 14a will be explained.

Provided within the first cleaning vessel 14a (second cleaning vessel 14b) are a forceps port 126a (126b) for establishing connection to the forceps channel opening 26 in the endoscope 12, an air insufflation or water purge port 128a (128b) for establishing connection to the air insufflation or water purge channel opening 28 in the endoscope 12, and a suction port 130a (130b) for establishing connection to the suction channel opening 30 in the endoscope 12. In the case of an endoscope having a forceps raising channel, a forceps raising port 124a (124b) is also provided to establish connection to the forceps raising channel opening.

Also provided within the first cleaning vessel 14a (second cleaning vessel 14b) are a detergent inlet 132a (132b) for introducing the detergent, a disinfectant inlet 134a (134b) for introducing the disinfectant, and a water supply inlet 136a (136b) for introducing tap water, as well as an air inlet 138a (138b) through which air is introduced for water leak detection, and a drain outlet 144a (144b).

The first cleaning vessel 14a is also provided with a level sensor 142a (142b) for detecting the volume of the fluid within the vessel, a thermometer TE for measuring the temperature of the fluid in the vessel, and a heater H for heating the fluid in the vessel.

The level sensor 142a (142b) may be exemplified by one that is capable of detecting the volume of the fluid at four levels; alternatively, four level sensors may be provided.

The forceps raising port 124a (124b) is connected via a valve 150a (150b) to valves 158a, 160a, and 162a (158b, 160b, and 160b); the forceps port 126a (126b) is connected to these valves via a valve 152a (152b); the air insufflation or water purge port 128a (128b) is connected to these valves via a valve 154a (154b); and the suction port 130a (130b) is connected to these valves via a valve 156a (156b).

Note that the valves to be used in the cleaner 10 are not limited in any particular way and any known valves that can be opened or closed automatically, such as electromagnetic valves or electrically operated valves, may be employed. It should, however, be mentioned that valves for draining the liquid waste from the cleaning vessels 14a and 14b or valves to be provided on lines (pipes) for returning the disinfectant to the disinfectant tank are preferably of an electrically operated type for such reasons as the smallness of the dead space within the valves.

The valve 158a (158b) is connected to an alcohol supply pump 110 associated with the alcohol tank 104.

The valve 160a (160b) is connected to the second air pump 116 for introducing air into the respective channels through the endoscope 12.

The valve 162a (162b) is connected to a water supply line 164 for supplying tap water to the relevant sites in the cleaner 10.

The water supply line 164 is connected to a drinking water faucet or the like for supplying tap water into the cleaner 10. As shown in FIG. 3, the water supply line 164 has, in order from the upstream end, a filter 166, a reducing valve 168, a first valve 170 and a second valve 172; the filter 166 serves to prevent contamination with foreign matter, and the reducing valve 168 serves to prevent undue pressure buildup in the piping system in the apparatus.

The pipe from the valve 162a (162b) is connected to somewhere between the first valve 170 and the second valve 172 on the water supply line 164. In the following description, this pipe which extends from the valve 162a (162b) to somewhere between the first valve 170 and the second valve 172 shall be referred to as a water supply pipe 163a (163b) for the sake of convenience. The water supply pipe 163a (163b) forms a branch in the middle of the way and this branch is connected to a circulating pump 182a (182b) to be described later that is associated with the first cleaning vessel 14a (second cleaning vessel 14b) and to a valve 180a (180b) also described later that is provided at the water supply inlet 136a (136b).

The second valve 172 is connected to the disinfectant tank 102 and to a valve 190a (190b) connected to the drain outlet 144a (144b) on the first cleaning vessel 14a (second cleaning vessel 14b).

The detergent inlet 132a (132b) is connected to the detergent pump 106-via a valve 176a (176b). The disinfectant inlet 134a (134b) is connected to the disinfectant pump 108 via a valve 178a (178b). The pipeline extending from the disinfectant tank 102 to the disinfectant inlet 134a (134b) on the cleaning vessel 14a (14b) combines with the disinfectant pump 108 and the valve 178a (178b) to constitute a disinfectant supply means in the present invention. The water supply inlet 136a (136b) on the other hand is connected to the water supply pipe 163a (136b) via the valve 180a (180b). In other words, the branch pipe that diverges from the water supply pipe 163a (163b) is connected to the valve 180a (180b), hence, to the water supply inlet 136a (136b).

The first cleaning vessel 14a (second cleaning vessel 14b) has the circulating pump 182a (182b) connected thereto. By means of the circulating pump 182a (182b), the fluid in the first cleaning vessel 14a (second cleaning vessel 14b) is supplied to the branch pipe that diverges from the water supply pipe 163a (163b) to extend to the valve 180a (180b), hence, to the water supply inlet 136a (136b).

The air inlet 138a (138b) through which air is introduced for water leak detection is connected via a valve 184a (184b) to a reducing valve 186 connected to the first air pump 114.

A pressure gage 188a (188b) is provided on the pipe extending from the air inlet 138a (138b) to the valve 184a (184b). Note that the pressure gage 188a (188b) is preferably a pressure transmitter or the like that delivers a signal to the first air pump 114 at the point in time when a predetermined pressure is reached.

The drain outlet 144a (144b) is connected to the water drain pump 118 via a valve 190a (190b).

The water drain pump 118 forces the fluid or the like within the cleaning vessels 14a and 14b to be sent to a drain line 194 having a valve 192. The water supply line 164 and the drain line 194 are connected via a bypass valve 196 in such a way that a site upstream of the filter 166 on the water supply line 164 is connected to a site upstream of the valve 192 on the drain line 194.

The pipe between the drain outlet 144a (144b) and the valve 190a (190b) forms a branch in the middle of the way, which is connected via a valve 198a (198b) to the second valve 172 on the water supply line 164 and to the disinfectant tank 102. The pipeline extending from the drain outlet 144a (144b) on the cleaning vessel 14a (14b) to the disinfectant tank 102 combines with the valve 198a (198b) to constitute a disinfectant recovery means in the present invention.

Described above is the general layout of the pipelines in the cleaner 10.

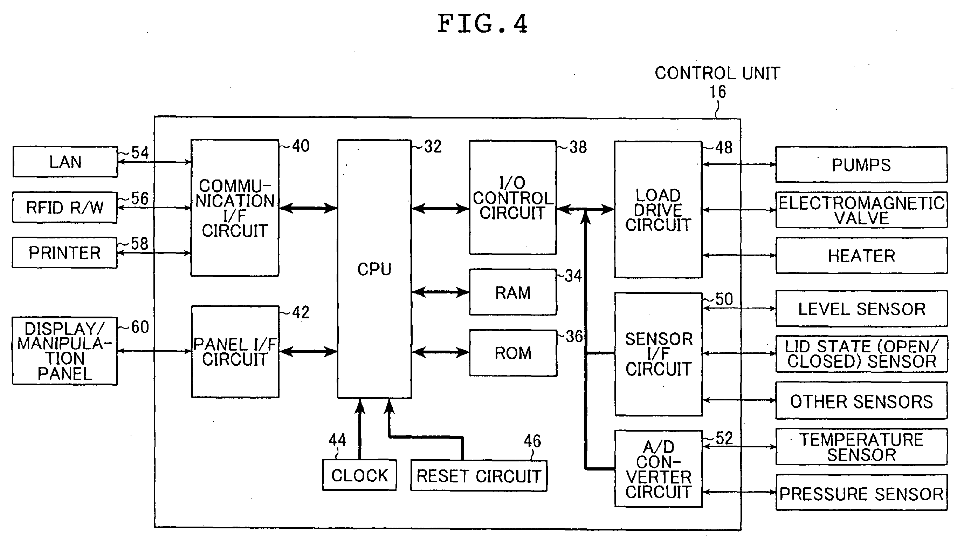

The control unit 16 controls the steps in the cleaning and disinfecting procedure to be performed in the first cleaning vessel 14a and the second cleaning vessel 14b. FIG. 4 is a block diagram showing in concept a schematic configuration of the control unit 16.

As shown in FIG. 4, the control unit 16 has a CPU 32, a RAM 234, a ROM 36, an I/O control circuit 38, a communication I/F circuit 40, a panel I/F circuit 42, a clock 44, a reset circuit 46, a load drive circuit 48, a sensor I/F circuit 50, and an A/D converter circuit 52.

The CPU 32 is for controlling the cleaning and disinfecting treatment as it is performed in the cleaner 10, and two cleaning vessels, the first cleaning vessel 14a and the second cleaning vessel 14b, are controlled by one CPU 32. Note that in the case where the cleaner 10 is equipped with three or more cleaning vessels, at least those cleaning vessels that share the same disinfectant tank are preferably controlled by one CPU 32 with regard to the cleaning and disinfecting treatment that is to be performed in those cleaning vessels.

The ROM 36 stores a variety of application programs including a program for controlling the cleaning and disinfecting treatment. The stored various application programs including the cleaning program are read from the ROM 36 by means of the CPU 32 and set in the RAM 34. The RAM 34 stores data on the history of cleaning and disinfecting operations previously performed by the cleaner 10. If desired, the ROM 36 may be so adapted as to store only the program for controlling the cleaning and disinfecting treatment that is associated with the number of the cleaning vessels the cleaner 10 has; in this case, the CPU 32 will always read that program from the ROM 36. Alternatively, the ROM 36 may store a plurality of programs for controlling the cleaning and disinfecting treatment that are associated with various vessel arrangements ranging from the use of one cleaning vessel to the use of a given number of cleaning vessels; in this case, the CPU 32 will select the program that is associated with the actual vessel arrangement the cleaner 10 has and will read that program from the ROM 36.

In another embodiment, program variations such as one that relates to the selection of a preferential cleaning mode (to be discussed later) may be stored in the ROM 36; in response to a command entered by the operator or following the choice it makes in association with the actual system configuration, the CPU 32 will choose the appropriate program and read it from the ROM 36.

The load drive circuit 48 drives the pumps (106, 108, 110, etc.), electromagnetic valves (150a, 152a, 154a, 156a, 198a, etc.) and the heater (H) that are shown in FIG. 3.

The sensor I/F circuit 50 is an interface for the level sensors (102L, 142a, 142b) that detect the fluid levels in the tanks and cleaning vessels, the sensors that detect the opening or closing of the lids on the cleaning vessels 14a and 14b, and any other sensors that are provided for the cleaner 10.

The A/D converter circuit 52 performs A/D conversion on the analog outputs from the temperature sensor (TE) and the pressure sensor (PE).

The communication I/F circuit 40 is a circuit for providing a communication interface with a LAN connector 54, a RFID R/W unit 56, and a printer 58.

The cleaner 10 uses the LAN connector 54 to have the control unit 16 connected to, for example, a network in a hospital for two-way communication of the data on the history of cleaning and disinfecting operations previously performed by the cleaner 10.

The RFID R/W unit 56 uses a RFID (radio-frequency identification system) to read or write the information about cleaning and disinfecting operations. For instance, the RFID R/W unit 56 can access an IC tag on the endoscope 12 to read the data on the history of cleaning operations the cleaner 10 has performed; alternatively, after cleaning and disinfection have been performed by the cleaner 10, the RFID R/W unit 56 can write the data on that treatment into the IC tag on the endoscope 12. Furthermore, the RFID R/W unit 56 can access an IC tag containing the information that identifies the operator who is responsible for cleaning the endoscope 12 and read that information out of this IC tag; alternatively, the RFID R/W unit 56 can write the history of cleaning operations the operator has executed into the operator's IC tag. In response to a control command issued from the CPU 32, the various kinds of data that have been read by the RFID R/W unit 56 out of the IC tag can be stored in the RAM 34 as data on the history of cleaning operations or they can be sent to the network via the LAN connector 54.

The printer 48 can print history management data. The printer 48 may be built in the cleaner 10 or it may be an external printer.

The panel I/F circuit 42 is an interface with a display/manipulation panel 60 on the cleaner 10. The display/manipulation panel 60 displays information about the cleaner 10 and it also functions as a touch panel on which the operator can enter commands.

We next describe how the endoscope 12 is cleaned and disinfected by the cleaner 10. Again, the following description focuses on the first cleaning vessel 14a but the second cleaning vessel 14b can be operated in entirely the same way to clean and disinfect the endoscope. It should also be understood that in the following description of the treatment that is performed by each of the steps involved, all valves except those which are described as being "opened" remain closed and that all pumps except those which are described as being "driven" remain off.

In the cleaner 10, the endoscope 12 is cleaned and disinfected by the basic steps of cleaning with a detergent, rinse, disinfecting with a disinfectant, and rinse in the order written.

First, the operator (technician) sets up the endoscope 12 at a specified position in the first cleaning vessel 14a; the operator then connects the forceps channel opening 26 on the endoscope 12 to the forceps port 124a, the air insufflation or water purge channel opening 28 to the air insufflation or water purge port 128a, and the suction channel opening 30 to the suction port 130a; if the endoscope 12 has a forceps raising channel, the operator also connects the forceps raising channel opening to the forceps raising port 124a.

Note that these connecting operations may be performed by known means commonly practiced on endoscope cleaners, for example, by using connectors or the like.

When the setting up of the endoscope 12 is completed and a command for the start of a cleaning operation is entered, the cleaner 10 first performs the cleaning step.

To begin with, the reducing valve 168 and the first valve 170 on the water supply line 164 as well as the valve 180a connecting to the water supply inlet 136 are opened so that a predetermined amount of tap water that flows through the water supply line 164 and the water supply pipe 163a is introduced into the first cleaning vessel 14a via the water supply inlet 136a (the introduction of tap water).

When the predetermined amount of tap water has been introduced, the valve 176a connecting to the detergent inlet 132a is opened and the detergent pump 106 is driven so that a predetermined amount of the detergent is supplied from the detergent tank 100 through the detergent inlet 132a into the first cleaning vessel 14a (the introduction of the detergent).

If necessary, following the introduction of tap water in the cleaning step, namely, at some point in time between the introduction of tap water and the introduction of the detergent, the step of water leak detection (to be described later) may be performed.

If no step of water leak detection is carried out, the introduction of tap water and the introduction of the detergent may be performed in parallel.

When the predetermined amounts of tap water and detergent have been introduced into the first cleaning vessel 14a, the valve 162a is opened, the circulating pump 182a is driven, and in an exemplary case, the valve 150a connecting to the forceps raising port 124a (only when the forceps raising channel opening is connected to the forceps raising port 124a), the valve 152a connecting to the forceps port 126a, the valve 154a connecting to the air insufflation or water purge port 128a, and the valve 156a connecting to the suction port 130a are opened sequentially one by one for a predetermined period of time. The durations for which the valves are opened may be the same or different for the respective ports. The detergent forced into the endoscope 12 from the channel openings 26, 28, 30, etc. that are at one end of the respective channels flows through those channels and emerge from the distal end of the insertion portion 24 (see FIG. 2), which is at the other end of each channel, to return into the first cleaning vessel 14a.

In this way, the detergent in the first cleaning vessel 14a is circulated through the individual channels in the endoscope 12 to clean them successively (the cleaning of the channels).

When the cleaning of the channels ends, the valve 180a connecting to the water supply inlet 136a is opened and the circulating pump 182 is driven.

As a result, the detergent circulates around the endoscope 12 within the first cleaning vessel 14a to clean the exterior of the endoscope 12 (the cleaning of the exterior with the flowing fluid).

When cleaning of the exterior is performed for a predetermined period of time, the valves 190a and 192 are opened and the drain pump 118 is driven to drain the detergent from within the first cleaning vessel 14a (the draining of the detergent).

When the first cleaning vessel 14a is completely drained of the detergent, the valve 160a is additionally opened, with the valves 190a and 192 remaining open, and the second air pump 116 is driven and, what is more, the valve 150a connecting to the forceps raising port 124a, the valve 152a connecting to the forceps port 126a, the valve 154a connecting to the air insufflation or water purge port 128a, and the valve 156a connecting to the suction port 130a are opened sequentially one by one.

In this way, air is forced into the individual channels through the endoscope 12 via the forceps raising port 124a, forceps port 126a, air insufflation or water purge port 128a, and the suction port 130a, whereupon the endoscope 12 is emptied of any detergent that is left within the respective channels (air insufflation as part of the cleaning step).

The above-described cleaning step may be performed more than once. If the cleaning step is to be.performed more than once, tap water and the detergent are again introduced into the first cleansing vessel 14a after the detergent is drained but before air insufflation is performed, so as to effect cleaning of the channels and cleaning of the exterior with the flowing fluid and the detergent is then drained. This procedure is repeated a predetermined number of times before air insufflation is finally performed.

The foregoing procedure completes the cleaning step and, subsequently, rinse is performed as a post-cleaning step.

The rinse as a post-cleaning step is performed in basically the same way as the above-described cleaning step except that introduction of the detergent into the first cleaning vessel 14a is not effected.

To state specifically, the reducing valve 168, the first valve 170 and the valve 180a are first opened so that a predetermined amount of tap water is introduced into the first cleaning vessel 14a (the introduction of tap water).

When the predetermined amount of tap water has been introduced into the first cleaning vessel 14a, the valve 162a is opened, the circulating pump 182a is driven, and the valves 150a, 152a, 154a and 156a are opened sequentially one by one, whereupon as in the cleaning of the channels, rinse of the channels is performed by rinsing the respective channels through the endoscope 12 with the tap water; thereafter, the valve 180a is opened and the circulating pump 182 is driven, whereupon as in the cleaning of the exterior with the flowing fluid, rinse of the exterior with the flowing fluid is performed by rinsing the exterior of the endoscope 12 with the tap water.

When rinse of the exterior with the flowing fluid ends, the valves 190a and 192 are opened and the drain pump 118 is driven to effect draining as in the draining after cleaning; then, the valve 160a is opened, the second air pump 116 is driven, and the valves 150a, 152a, 154a and 156a are opened sequentially one by one, whereupon as in the air insufflation after cleaning, air insufflation as part of the rinse step is performed to complete the rinse as a post-cleaning step.

The above-described rinse step may be performed more than once. If the rinse step is to be performed more than once, tap water is again introduced into the first cleansing vessel 14a after the rinse water is drained but before air insufflation is performed, so as to effect rinse of the channels and rinse of the exterior with the flowing fluid. This procedure is repeated a predetermined number of times before air insufflation is finally performed.

The foregoing procedure completes the rinse as a post-cleaning step and, subsequently, the disinfecting step is performed.

In the disinfecting step, the valve 178a connecting to the disinfectant inlet 134a is first opened and the disinfectant pump 108 is driven to introduce a predetermined amount of the disinfectant into the first cleaning vessel 14a (the introduction of the disinfectant).

When the predetermined amount of the disinfectant has been introduced into the first cleaning vessel 14a, the interiors of the respective channels through the endoscope 12 are disinfected as in the above-described cleaning of the channels.

To state specifically, the valve 162a is opened and the circulating pump 182a is driven and, what is more, the valves 150a, 152a, 154a and 156a that are connected to the ports connecting to the respective channels through the endoscope 12 are opened sequentially one by one for a specified period of time.

As a result, the disinfectant in the first cleaning vessel 14a is circulated through the individual channels in the endoscope 12 to disinfect those channels (the disinfection of the channels).

When the disinfection of the channels ends, the exterior of the endoscope 12 is disinfected as in the above-described cleaning of the exterior with the flowing fluid.

To state specifically, the valve 180a connecting to the water supply inlet 136a is opened and the circulating pump 182 is driven so that the disinfectant in the first cleaning vessel 14a is circulated around the endoscope 12 to disinfect its exterior with the disinfectant (the disinfection of the exterior with the flowing fluid).

Note here that if the disinfectant can be spread into every part of the interior and the exterior of the endoscope 12 without circulating it, the disinfection of the channels and the disinfection of the exterior can be effected by immersing the endoscope 12 in the disinfectant after it has been spread into the necessary areas.

When the disinfection of the exterior with the flowing fluid has been conducted for a specified period of time, the valve 198a connecting to the drain outlet 144a is opened so that the disinfectant is returned into the disinfectant tank 102 (the recovery of the disinfectant).

In the illustrated case of the cleaner 10, a pump or the like is not used to recover the disinfectant but it is dropped under its own weight to return into the disinfectant tank 102.

When the disinfectant in the first cleaning vessel 14a has been recovered into the disinfectant tank 102, air is insufflated into the respective channels through the endoscope 12 as in the above-described air insufflation as part of the cleaning step.

To state specifically, the valve 160a is opened and the second air pump 116 is driven and, what is more, the valves 150a, 152a, 154a and 156a are opened sequentially one by one. In this way, air is forced into the individual channels through the endoscope 12 via the forceps raising port 124a, forceps port 126a, air insufflation or water purge port 128a, and the suction port 130a, whereupon the endoscope 12 is emptied of any disinfectant that is left within the respective channels (air insufflation as part of the disinfecting step).

The foregoing procedure completes the disinfecting step and, subsequently, rinse is performed as a post-disinfecting step.

The rinse as a post-disinfecting step is performed in basically the same way as the above-described rinse as a post-cleaning step.

To state specifically, the reducing valve 168, the first valve 170 and the valve 180a are first opened so that a predetermined amount of tap water is introduced into the first cleaning vessel 14a (the introduction of tap water).

When the introduction of tap water ends, the valve 162a is opened, the circulating pump 182a is driven, and the valves 150a, 152a, 154a and 156a are opened sequentially one by one for a specified period of time so that rinse of the channels is performed by rinsing the respective channels through the endoscope 12 with the tap water. Subsequently, the valve 180a is opened and the circulating pump 182 is driven so that rinse of the exterior with the flowing fluid is performed by rinsing the exterior of the endoscope 12 with the tap water.

When rinse of the exterior with the flowing fluid ends, the valves 190a and 192 are opened and the drain pump 118 is driven to effect draining as part of the rinse step. Thereafter, the valve 160a is opened, the second air pump 116 is driven, and the valves 150a, 152a, 154a and 156a are opened sequentially one by one so that air insufflation as part of the rinse step is performed to complete the rinse as a post-disinfecting step.

The rinse as a post-disinfecting step may be performed more than once as in the rinse as a post-cleaning step.

Ending of the rinse step after the disinfecting step completes the cleaning of the endoscope 12 with the cleaner 10 and a visual display, an audible alarm or the like may be used to inform the operator that the cleaning of the endoscope 12 has ended.

As already mentioned, the cleaner 10 has the tanks, pumps and many other parts shared by the first cleaning vessel 14a and the second cleaning vessel 14b. However, except for the supply systems for the detergent and the like, the water supply line 164 and the drain line 194, the two cleaning vessels have mutually independent pipelines, so they can be operated to perform the same treatment at the same time or perform different treatments at the same time (the two vessels perform asynchronous treatments).

While the basic procedure of cleaning the endoscope 12 with the cleaner 10 has been described above, the cleaner 10 is capable of performing various treatments other than the above-described cleaning process.

For example, alcohol flushing may optionally be performed to ensure that the cleaned interiors of the respective channels through the endoscope 12 are dried at an accelerated speed.

Alcohol flushing is performed in the following manner; after the cleaning step ends, the valve 158a is opened and the alcohol pump 110 is driven, and what is more, the valve 150a connecting to the forceps raising port 124a, the valve 152a connecting to the forceps port 126a, the valve 154a connecting to the air insufflation or water purge port 128a, and the valve 156a connecting to the suction port 130a are opened sequentially one by one for a predetermined period of time.

Subsequently, as in the air insufflation conducted in each of the steps described above, the valve 160a is opened and the second air pump 116 is driven and, what is more, the valves 150a, 152a, 154a and 156a are opened sequentially one by one so that air is insufflated into the respective channels through the endoscope 12 to purge them of the alcohol and dry their interiors.

In addition, the drain outlet 144a and the valves 190a and 192 are opened and the drain pump 118 is driven to purge the first cleaning vessel 14a of the alcohol that has been drained into that vessel.

If another embodiment, the cleaner 10 may be so operated as to perform self-disinfection in which the water supply line 164, the drain line 194 and the like are disinfected with the disinfectant.

In this self-disinfecting step, the valve 178a connecting to the disinfectant inlet 134a is first opened and the disinfectant pump 108 is driven to introduce a predetermined amount of the disinfectant into the first cleaning vessel 14a.

Subsequently, the valve 190a connecting to the drain outlet 144a, the bypass valve 196, the reducing valve 168, the first valve 170, and the valve 180a connecting to the water supply inlet 136a are opened and the drain pump 118 is driven to circulate the disinfectant through the path including the water supply line 164 and the drain line 194.

In the illustrated case of the cleaner 10, a preferred embodiment is such that when the process of self-disinfection ends, the cleaner 10 is drained of the disinfectant and the disinfectant tank 102 is refilled with a fresh disinfectant.

To state specifically, when the disinfectant has been circulated for a specified period of time through the aforementioned path including the water supply line 164 and the drain line 194, the valves 190a and 192 are opened and the drain pump 118 is driven to drain the disinfectant. In addition, the valve 178a is opened and the disinfectant pump 108 is driven so that any disinfectant that might stay within the disinfectant tank 102 is drained off by being transferred into the first cleaning vessel 14a.

When all of the disinfectant in the cleaner 10 has been drained, the reducing valve 168, the first valve 170 and the second valve 172 are opened and a predetermined amount of tap water is charged into the disinfectant tank 102. Subsequently, the operator installs a bottle of disinfectant B on each of the two attachments 102. The disinfectant may be introduced into the disinfectant tank 102 under its own weight, whereupon the disinfectant tank 102 is refilled with the fresh disinfectant.

As already mentioned, after the introduction of tap water in the cleaning step, the cleaner 10 may optionally be so operated as to perform detection of any water leakage from the individual channels through the endoscope 12.

If the step of water leak detection is to be carried out, the endoscope 12 to be cleaned is set up in the first cleaning vessel 14a and at the same time the air inlet 138a (138b) is connected to the pressurizing opening 31 for leak detection that is provided on the endoscope 12 (see FIG. 2). When the introduction of tap water in the cleaning step ends, the first air pump 114 is driven and the reducing valve 186 as well as the valve 184a are opened. At the point in time when the pressure gage 188a reads a predetermined pressure, the drive of the first air pump 144 is stopped. This is preferably done automatically in response to a signal the pressure gage 188a delivers to the air pump 114 in accordance with the result of pressure measurement.

When the pressurization ends, the endoscope 12 is visually checked for any air bubbles that might come out of it; if any air bubbles are seen coming out, any one of the channels through the endoscope 12 might have a leak, so the cleaning of the endoscope 12 is suspended at that point of time. Alternatively, if the pressure measured with the pressure gage 188a drops below a specified level within a specified period of time, any one of the channels through the endoscope 12 might again have a leak, so the cleaning of the endoscope 12 is suspended at that point of time. The pressure gage 188a may be so adapted that at the time when the pressure it measures drops below a specified level, it issues a warning to the effect that any one of the channels through the endoscope 12 has a leak.

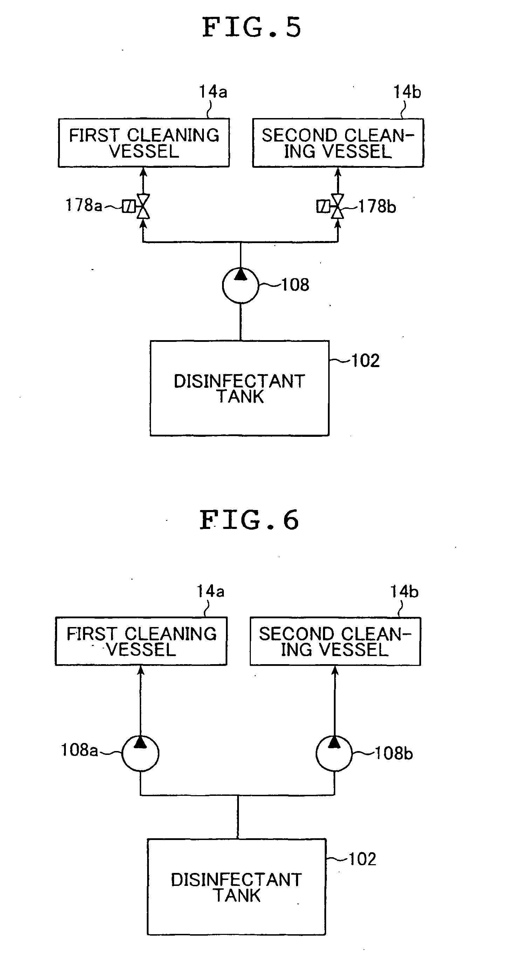

FIG. 5 shows diagrammatically a schematic arrangement of the first cleaning vessel 14a, the second cleaning vessel 14b, and the disinfectant tank 102.

The cleaner 10 in the first embodiment is such that the disinfectant tank 102 has a capacity not smaller than the total volume of the disinfectant that is required by the step of disinfection to be performed in the first.cleaning vessel 14a and the second cleaning vessel 14b. As already mentioned before, the cleaner 10 is equipped with a single disinfectant pump 108 for supplying the disinfectant from the disinfectant tank 102 and the pipeline diverges at a point downstream of the disinfectant pump 108 to form branches which communicate with the first cleaning vessel 14a and the second cleaning vessel 14b via the valves 178a and 178b, respectively.

Because of this configuration, the cleaner 10 has the advantage that the disinfectant can be managed in an integrated fashion. As another advantage, the two cleaning vessels 14a and 14b share one disinfectant tank 102, so compared to the case where the two cleaning vessels are equipped with different disinfectant tanks and associated supply systems, the apparatus can be configured in a compact way, which contributes to space saving. Further in addition, the first cleaning vessel 14a and the second cleaning vessel 14b can be filled with the disinfectant simultaneously, so the two cleaning vessels 14a and 14b can execute the cleaning step simultaneously. The disinfectant can be supplied into the two cleaning vessels 14a and 14b simultaneously; however, if this is done, the time it takes is twice as long and the efficiency is lowered, so the step of supplying the disinfectant preferably does not overlap between the two cleaning vessels 14a and 14b.

Each of the steps in the cleaning and disinfecting procedure to be followed by the cleaner 10 is controlled by the control unit 16. The control unit 16 depends on the CPU 32 for reading a preset cleaning and disinfecting program out of the ROM 36 and, in accordance with that program, controls the pumps, valves, sensors and other components in the cleaner 10 to execute each of the steps in the cleaning and disinfecting procedure. In this case, in order to ensure that the first cleaning vessel 14a and the second cleaning vessel 14b which share the disinfectant tank 102 will not be supplied with the disinfectant simultaneously, the control unit 16 controls the cleaning and disinfecting steps in the two vessels in such a way that the they are supplied with the disinfectant at different timings.

The cleaner 10 can also be set for a preferential cleaning mode that allows either one of the two cleaning vessels 14a and 14b to execute the cleaning and disinfecting treatment in preference to the other vessel.

In order to supply the disinfectant into the two cleaning vessels simultaneously without lowering the efficiency in the design depicted in FIG. 5, one may use a disinfectant pump having twice the ability to supply the disinfectant.

The first embodiment may be modified as shown in FIG. 6, where the cleaner 10 uses two disinfectant pumps 108a and 108b that supply the disinfectant into the first cleaning vessel 14a and the second cleaning vessel 14b, respectively, from the disinfectant tank 102. This design also allows the disinfectant to be supplied into the two cleaning vessels simultaneously.

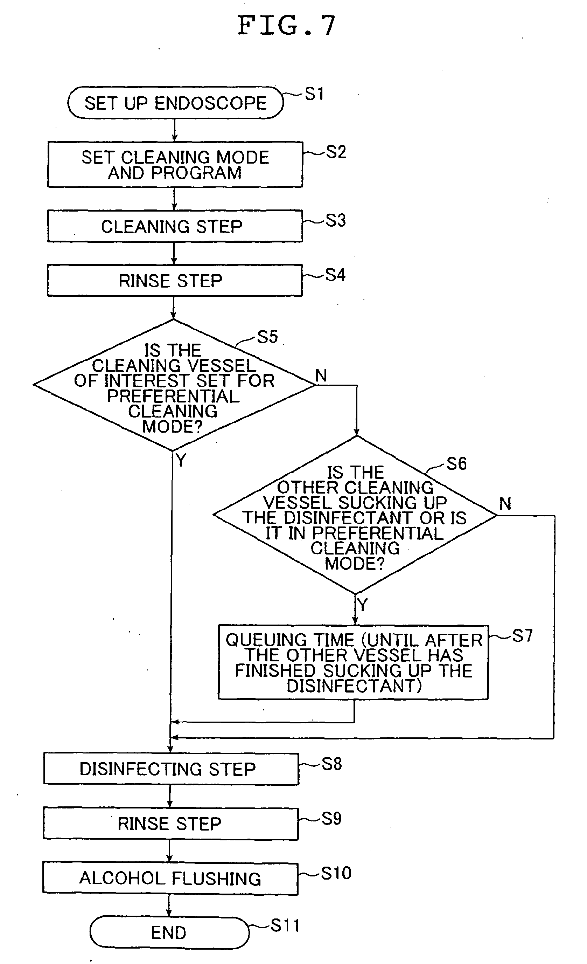

FIG. 7 is a flow sheet showing an example of a cleaning and disinfecting treatment according to the first embodiment of the present invention.

In step S1, the endoscope 12 is set up in the first cleaning vessel 14a or the second cleaning vessel 14b. Then, in step S2, the control unit 16 depends on the CPU 32 for setting the cleaning mode, reading the associated cleaning program out of the ROM 36, and setting this program in the RAM 34. The following description concerns the case where the endoscope 12 is set up in the first cleaning vessel 14a.

The cleaning mode may be a normal cleaning mode or a preferential cleaning mode. In the normal cleaning mode, the cleaning and disinfecting treatment is executed in the order in which the endoscope 12 was set up in the cleaning vessels 14a and 14b. In the preferential cleaning mode, the cleaning vessel accommodating the endoscope 12 that needs to be cleaned quickly is allowed to execute the cleaning and disinfecting treatment in preference to the other cleaning vessel.

The cleaning mode to be executed can.be designated by the operator from the display/manipulation panel 60 (see FIG. 4) provided on the cleaner 10. In response to an input signal from the display/manipulation panel 60, the control unit 16 sets the designated cleaning mode (step S2).

Then, in step S3, the control unit 16 executes the above-described cleaning step and, in subsequent step S4, executes the above-described rinse step as a post-cleaning step.

When the rinse step S4 ends, the process goes to step S5, where the control unit 16 asks whether the cleaning job currently done in the first cleaning vessel 14a is what has been set for the preferential cleaning mode.

If the answer is affirmative, a decision for YES (Y) is made in step S5 and the process goes to the disinfecting step S8. In other words, the cleaner 10 continues the cleaning step in the first cleaning vessel 14a irrespective of what is being done in the second cleaning vessel 14b.

If the answer is negative, a decision for NO (N) is made in step S5 and the process goes to step S6.

In step S6, the control unit 16 asks whether the other vessel (the second cleaning vessel 14b in the case under consideration) is sucking up the disinfectant and also asks whether the other vessel has been set for the preferential cleaning mode. If the other vessel (the second cleaning vessel 14b) is not sucking up the disinfectant, nor is it in the preferential cleaning mode, a decision for NO is made in step S6 and the process goes to step S8. If the other vessel is sucking up the disinfectant or it has been set for the preferential cleaning mode, the process goes to step S7, creating a queuing time that lasts until the other vessel finishes sucking up the disinfectant and in the meantime the cleaning and disinfecting treatment in the first cleaning vessel 14a is interrupted. When the queuing time in step S7 ends, the process goes to step S8.

In step S8, the above-described disinfecting step is executed. When the disinfecting step ends, rinse as a post-disinfecting step is executed in step S9. If alcohol flushing is to be done, the step of alcohol flushing is executed in step S10, whereupon the cleaning and disinfecting treatment is completed (step S11).

According to the cleaning method described above, the disinfectant can be sucked up at different timings in the two cleaning vessels, so the endoscope 12 that was the first to enter the disinfecting step can be cleaned without suffering a drop in efficiency and, what is more, the vessel that has been set for the preferential cleaning mode can be subjected to the disinfecting step in preference over the other vessel.

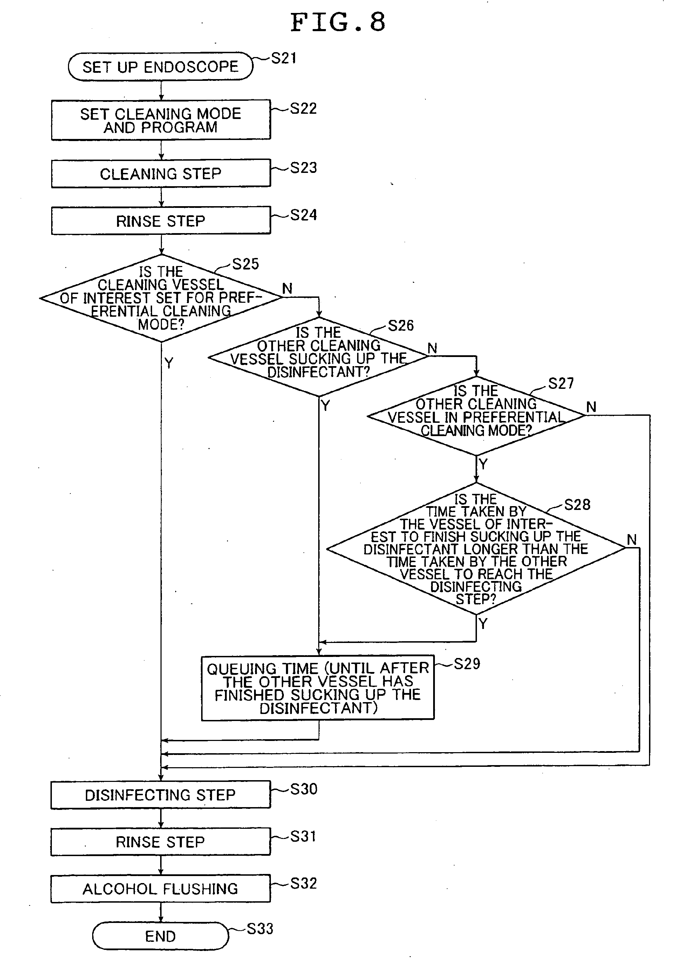

FIG. 8 is a flow sheet showing another example of the cleaning and disinfecting treatment according to the first embodiment of the present invention.

In the case illustrated in FIG. 7, the supply of the disinfectant into the cleaning vessel of interest is preceded by the supply of the disinfectant into the other cleaning vessel whenever the latter is set for the preferential cleaning mode. This is not the case for FIG. 8 and even if the other cleaning vessel has been set for the preferential cleaning mode, the cleaning and disinfecting treatment being performed in the vessel of interest is not interrupted but the process enters the disinfecting step if the supply of the disinfectant into the vessel of interest is to complete before the disinfectant is supplied into the other vessel.

In the flow sheet of FIG. 8, steps S21 to S25 are the same as steps S1 to S5 in the flow sheet of FIG. 7.

If a vessel of interest (say, the first cleaning vessel 14a) has been set for the preferential cleaning mode, a decision for YES is made in step S25 and the process jumps to step S30 and enters the disinfecting step. If the vessel of interest is not in the preferential cleaning mode, a decision for NO is made in step S25 and the process goes to step S26.

In step S26, the control unit 16 asks whether the other vessel (say, the second cleaning vessel 14b) is sucking up the disinfectant. If the answer is affirmative, the process goes to step S29, creating a queuing time that lasts until the other vessel finishes sucking up the disinfectant and in the meantime the cleaning and disinfecting treatment in the vessel of interest is interrupted.

If the answer is negative, the process goes to step S27 and the control unit 16 asks whether the other vessel has been set for the preferential cleaning mode. If the answer is affirmative, the process goes to step S28; otherwise, the process goes to step S30 and enters the disinfecting step.

In step S28, the time it takes for the other vessel to reach the disinfecting step is compared with the time it takes for the vessel of interest to complete the sucking up of the disinfectant and the control unit 16 asks if the former is longer than the latter. To state specifically, in the case where the other vessel that was set for the preferential cleaning mode has started the cleaning step after the vessel of interest to allow for a sufficient time before the other vessel enters the disinfecting step and starts to suck up the disinfectant so that the sucking up of the disinfectant in the vessel of interest can be completed within that period of time, a decision for NO (the time for the vessel of interest to be supplied with the disinfectant ≤ the time for the other vessel to reach the disinfecting step) is made in step S28 and the process goes to the disinfecting step S30. On the other hand, if the calculation shows that the other vessel will reach the disinfecting step before the supply of the disinfectant into the vessel of interest ends, a decision for YES (the time for the vessel of interest to be supplied with the disinfectant > the time for the other vessel to reach the disinfecting step) is made in step S28 and the process goes to step S29, creating a queuing time that lasts until the other vessel finishes sucking up the disinfectant and in the meantime the cleaning and disinfecting treatment in the vessel of interest is interrupted. When the other vessel finishes sucking up the disinfectant and the queuing time in step S29 ends, the process enters the disinfecting step S30.

The subsequent steps S30 to S33 are executed in the same manner as steps S8 to S11 in the case shown in FIG. 7.

According to the method just described above, the advantage of the flow sheet of FIG. 7 is of course obtained and, in addition, if the other vessel is in the preferential cleaning mode but there is a sufficient time difference for the vessel of interest to perform the intended treatment before the other vessel, the cleaning job in the vessel of interest can be allowed to proceed and this contributes to an enhanced cleaning efficiency.

We next describe the second embodiment of the present invention.

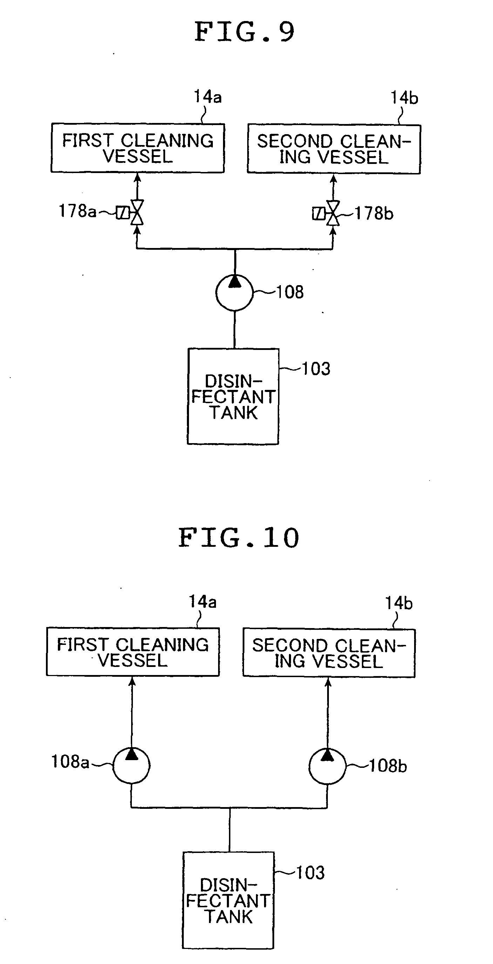

FIG. 9 shows diagrammatically a schematic arrangement of the first cleaning vessel 14a, the second cleaning vessel 14b, and a disinfectant tank 103.

In the second embodiment, the disinfectant tank 103 has substantially the same capacity as the volume of the disinfectant that is required in the first cleaning vessel 14a. Since the first cleaning vessel 14a and the second cleaning vessel 14b in the cleaner 10 have the same configuration, they require the same volume of the disinfectant.

Note that the capacity of the disinfectant tank 103 should not be smaller than whichever the greater of the volumes of the disinfectant that is required in the first and second cleaning vessels 14a and 14b but that it should be smaller than the total volume of the disinfectant that is required in the two vessels.

With this design, the cleaner 10 can be configured in a more compact way, contributing to greater space saving than in the case shown in FIG. 5. In this design, the control unit 16 controls the cleaning and disinfecting procedure in the first cleaning vessel 14a and the second cleaning vessel 14b in such a way that the disinfecting step does not overlap between the two vessels.

The second embodiment may be modified as shown in FIG. 10, where the cleaner 10 employs two disinfectant pumps 108a and 108b that supply the disinfectant into the first cleaning vessel 14a and the second cleaning vessel 14b, respectively, from the disinfectant tank 103.

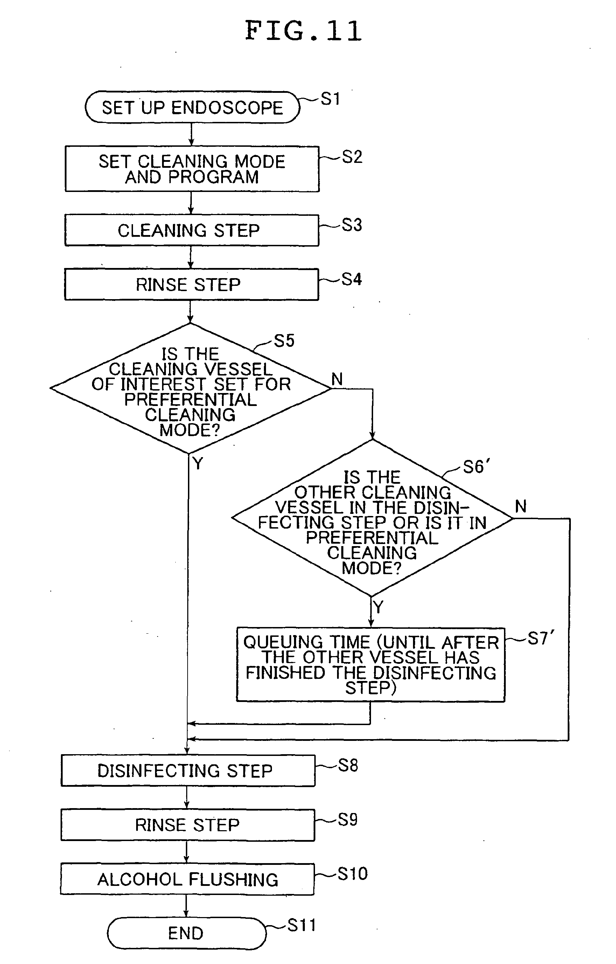

FIG. 11 is a flow sheet showing an example of the cleaning and disinfecting treatment according to the second embodiment of the present invention. Steps S1 to S5 and steps S8 to S11 in this flow sheet are the same as in the flow sheet of FIG. 7.

To state specifically, step S1 consists of setting up the endoscope 12 in the first cleaning vessel 14a or the second cleaning vessel 14b; then, in step S2, an applicable cleaning mode and the associated cleaning program are set. In accordance with the cleaning program thus set, the cleaning step is executed in step S3 and the rinse step in step S4.

Then, in step S5, the control unit 16 asks whether the vessel of interest (say, the first cleaning vessel 14a) has been set for the preferential cleaning mode; if the answer is affirmative, the process jumps to step S8 and, if not, goes to step S6'.

In step S6', the control unit 16 asks whether the other vessel (say, the second cleaning vessel 14b) is executing the disinfecting step and also asks whether the other vessel has been set for the preferential cleaning mode. If the other vessel is not in the disinfecting step, nor is it in the preferential cleaning mode, a decision for NO is made in step S6' and the process goes to step S8. If the other vessel is in the disinfecting step or it has been set for the preferential cleaning mode, the process goes to step S7', creating a queuing time that lasts until the other vessel goes through the disinfecting step and in the meantime the cleaning and disinfecting treatment in the vessel of interest is interrupted. When the queuing time in step S7' ends, the process goes to step S8.

In step S8, the above-described disinfecting step is executed. When the disinfecting step ends, the rinse step is executed in step S9. If alcohol flushing is to be done, the step of alcohol flushing is executed in step S10, whereupon the cleaning and disinfecting treatment is completed (step S11).

According to the cleaning method just described above, the disinfecting step does not overlap between the two cleaning vessels but the disinfectant in the disinfectant tank 103 is first supplied into one cleaning vessel and after the disinfecting step ends and the disinfectant is recovered into the disinfectant tank 103, the same disinfectant can be supplied into the other cleaning vessel. As a result, even the disinfectant tank 103 whose capacity is no more than the capacity of one cleaning vessel is capable of sequential cleaning in the two cleaning vessels. What is more, the cleaning vessel that has been set for the preferential cleaning mode can perform the cleaning and disinfecting of the endoscope 12 in preference over the other vessel.

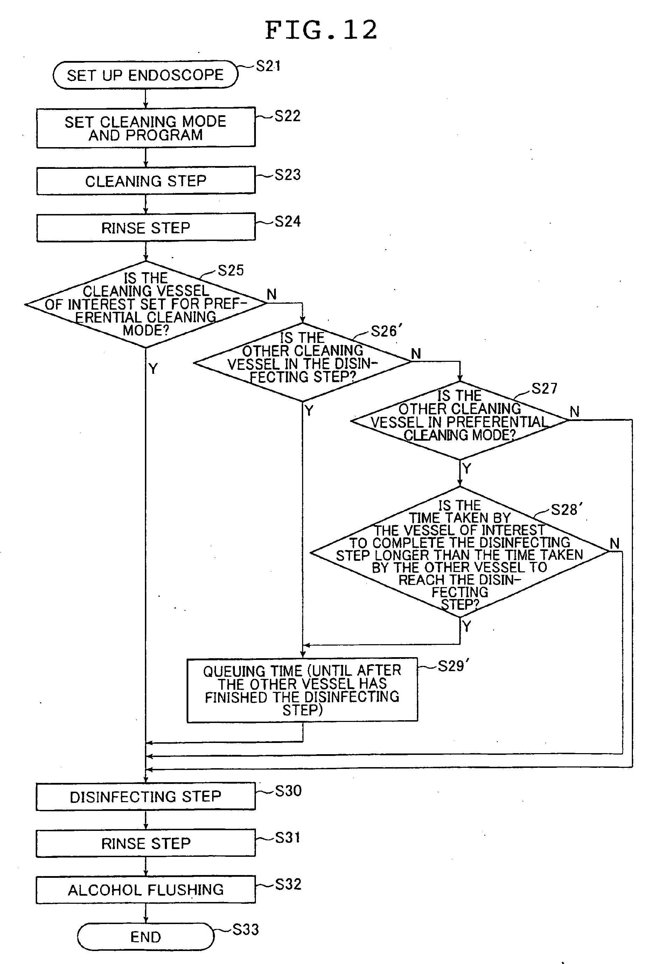

FIG. 12 is a flow sheet showing another example of the cleaning and disinfecting treatment according to the second embodiment of the present invention.

In the case illustrated in FIG. 11, the supply of the disinfectant into the cleaning vessel of interest is preceded by the supply of the disinfectant into the other cleaning vessel whenever the latter is set for the preferential cleaning mode. This is not the case for FIG. 12 and even if the other cleaning vessel has been set for the preferential cleaning mode, the cleaning and disinfecting treatment being performed in the vessel of interest is not interrupted but the disinfecting step is executed if the disinfecting step in the vessel of interest is to complete before the other vessel enters the disinfecting step.

In the flow sheet of FIG. 12, steps S21 to S25 are the same as steps S1 to S5 in the flow sheet of FIG. 11.

If a vessel of interest (say, the first cleaning vessel 14a) has been set for the preferential cleaning mode, a decision for YES is made in step S25 and the process jumps to step S30 and enters the disinfecting step. If the vessel of interest is not in the preferential cleaning mode, a decision for NO is made in step S25 and the process goes to step S26'.

In step S26', the control unit 16 asks whether the other vessel (say, the second cleaning vessel 14b) is in the disinfecting step. If the answer is affirmative, the process goes to step S29', creating a queuing time that lasts until the other vessel finishes the disinfecting step and in the meantime the cleaning and disinfecting treatment in the vessel of interest is interrupted.

If the answer is negative, the process goes to step S27 and the control unit 16 asks whether the other vessel has been set for the preferential cleaning mode. If the answer is affirmative, the process goes to step S28'; otherwise, the process goes to step S30 and enters the disinfecting step.

In step S28', the time it takes for the other vessel to reach the disinfecting step is compared with the time it takes for the vessel of interest to finish the disinfecting step and the control unit 16 asks if the former is longer than the latter. To state specifically, in the case where the other vessel that was set for the preferential cleaning mode has started the cleaning step after the vessel of interest to allow for a sufficient time before the other vessel enters the disinfecting step so that the step of disinfection in the vessel of interest can be completed and the disinfectant returned into the disinfectant tank 103 within that period of time, a decision for NO (the time for the vessel of interest to complete the disinfecting step ≤ the time for the other vessel to reach the disinfecting step) is made in step S28' and the process goes to the disinfecting step S30. On the other hand, if the calculation shows that the other vessel will reach the disinfecting step before the vessel of interest finishes the disinfecting step, a decision for YES (the time for the vessel of interest to complete the disinfecting step > the time for the other vessel to reach the disinfecting step) is made in step S28' and the process goes to S29', creating a queuing time that lasts until the other vessel finishes the disinfecting step and in the meantime the cleaning and disinfecting treatment in the vessel of interest is interrupted. When the other vessel completes the disinfecting step and the queuing time in step S29' ends, the process enters the disinfecting step S30.

The subsequent steps S30 to S33 are executed in the same manner as steps S8 to S11 in the case shown in FIG. 11.

According to the method just described above, the advantage of the flow sheet of FIG. 11 is of course obtained and, in addition, if the other vessel is in the preferential cleaning mode but there is a sufficient time difference for the vessel of interest to perform the intended treatment before the other vessel, the cleaning job in the vessel of interest can be allowed to proceed and this contributes to an enhanced cleaning efficiency.

While the endoscope cleaner of the present invention has been described in detail on the foregoing pages, the present invention is by no means limited to the embodiments described above and it should be understood that various improvements and alterations can be made without departing from the scope of the present invention.

## Claims

1. An endoscope cleaner (10) comprising:
a plurality of cleaning vessels (14a,b) each of which can accommodate an endoscope (12);
at least one disinfectant tank (102) that holds a disinfectant, a first number of the at least one disinfectant tank (102) being less than a second number of the plurality of cleaning vessels (14a,b);
disinfectant supply means for supplying the disinfectant from the at least one disinfectant tank (102) to the plurality of cleaning vessels (14a,b);
disinfectant recovery means (198a) for recovering the disinfectant from the plurality of cleaning vessels (14a,b) to the at least one disinfectant tank (102); and
a control unit (16) that controls the disinfectant supply means and the disinfectant recovery means to control steps of a cleaning and disinfecting treatment to be performed in the plurality of cleaning vessels (14a,b);
**characterized in that** one of the at least one disinfectant tank (102) has a capacity not smaller than a volume of the disinfectant required to perform a disinfecting step in one of the plurality of cleaning vessels (14a,b) and the at least one disinfectant tank has a total capacity which is less than a total volume of the disinfectant required to perform the disinfecting step in all of the plurality of cleaning vessels (14a,b).

2. The endoscope cleaner according to claim 1, wherein the first number of the at least one disinfectant tank is unity.

3. The endoscope cleaner according to claim 1 or 2, wherein the second number of the plurality of cleaning vessels (14a,b) is two.

4. The endoscope cleaner according to any one of claims 1 to 3, wherein the control unit controls the steps of the cleaning and disinfecting treatment in two or more cleaning vessels that share a specific disinfectant tank of the at least one disinfectant tank such that the two or more cleaning vessels will not execute the disinfecting step simultaneously.

5. The endoscope cleaner according to any one of claims 1 to 4, wherein, before a cleaning vessel in the two or more cleaning vessels that share the specific disinfectant tank starts a disinfecting step, the control unit checks if a remainder of the two or more cleaning vessels is in the disinfecting step and, if it is in the disinfecting step, causes the cleaning vessel not to start the disinfecting step until after the disinfecting step in the remainder of the two or more cleaning vessels is completed and, if it is not in the disinfecting step, causes the cleaning vessel to start the disinfecting step.

6. The endoscope cleaner according to any one of claims 1 to 5, wherein the control unit checks if the two or more cleaning vessels that share the specific disinfectant tank have been set for a preferential cleaning mode and controls the steps of the cleaning and disinfecting treatment in the two or more cleaning vessels such that the disinfecting step in other cleaning vessel than a specific cleaning vessel that has been set for the preferential cleaning mode will be started after the disinfecting step in the specific cleaning vessel that has been set for the preferential cleaning mode is completed.

## Patentansprüche

1. Endoskopreiniger (10) umfassend:
mehrere Reinigungsbehälter (14a,b), von denen jeder ein Endoskop aufnehmen kann;
mindestens einen Desinfektionsmitteltank (102), der ein Desinfektionsmittel aufnimmt, wobei eine erste Anzahl des mindestens einen Desinfektionsmitteltanks (102) geringer ist als eine zweite Anzahl der mehreren Reinigungsbehälter (14a,b);
eine Desinfektionsmittel-Zuführeinrichtung zum Zuführen des Desinfektionsmittels aus dem mindestens einen Desinfektionsmitteltank (102) zu den mehreren Reinigungsbehältern (14a,b);
eine Desinfektionsmittel-Rückführeinrichtung (198a) zum Zurückführen des Desinfektionsmittels aus den mehreren Reinigungsbehältern (14a,b) zu dem mindestens einen Desinfektionsmitteltank (102); und
eine Steuereinheit (16), die die Desinfektionsmittel-Zuführeinrichtung und die Desinfektionsmittel-Rückführeinrichtung steuert, um die Schritte einer Reinigungs- und Desinfektionsbehandlung zu steuern, die in den mehreren Reinigungsbehältern (14a,b) auszuführen sind;
**dadurch gekennzeichnet, dass** der mindestens eine Desinfektionsmitteltank (102) eine Kapazität besitzt, die nicht kleiner ist, als ein zum Ausführen eines Desinfektionsschritts in einem der mehreren Reinigungsbehälter (14a,b) erforderliches Desinfektionsmittel-Volumen, und der mindestens eine Desinfektionsmitteltank eine Gesamtkapazität besitzt, die geringer ist als ein Gesamtvolumen von Desinfektionsmittel, welches erforderlich ist zur Ausführung des Desinfektionsschritts in sämtlichen der mehreren Reinigungsbehälter (14a,b).

2. Endoskop-Reiniger nach Anspruch 1, bei dem die erste Anzahl des mindestens einen Desinfektionsmitteltanks eins beträgt.

3. Endoskop-Reiniger nach Anspruch 1 oder 2, bei dem die zweite Anzahl der mehreren Reinigungsbehälter (14a,b) zwei ist.

4. Endoskop-Reiniger nach einem der Ansprüche 1 bis 3, bei dem die Steuereinheit die Schritte der Reinigungs- und Desinfektionsbehandlung in zwei oder mehr Reinigungsbehältern steuert, die einen spezifischen Desinfektionsmitteltank von dem mindestens einen Desinfektionsmitteltank gemeinsam nutzen, so dass die zwei oder mehr Reinigungsbehälter den Desinfektionsschritt nicht gleichzeitig ausführen.

5. Endoskop-Reiniger nach einem der Ansprüche 1 bis 4, bei dem, bevor ein Reinigungsbehälter von den zwei oder mehr Reinigungsbehältern, die einen spezifischen Desinfektionsmitteltank gemeinsam benutzen, einen Desinfektionsschritt startet, die Steuereinheit prüft, ob sich ein Rest von den zwei oder mehr Reinigungsbehältern im Desinfektionsschritt befindet, und, falls er sich in dem Desinfektionsschritt befindet, den Reinigungsbehälter dazu bringt, den Desinfektionsschritt nicht eher einzuleiten, als bis der Desinfektionsschritt des Rests von den zwei oder mehr Reinigungsbehältern abgeschlossen ist, und falls er sich nicht in dem Desinfektionsschritt befindet, den Reinigungsbehälter veranlasst, den Desinfektionsschritt zu beginnen.

6. Endoskop-Reiniger nach einem der Ansprüche 1 bis 5, bei dem die Steuereinheit prüft, ob die zwei oder mehr Reinigungsbehälter die den spezifischen Desinfektionstank gemeinsam benutzen, für einen Vorzugs-Reinigungsmodus eingerichtet wurden, und die Schritte der Reinigungs- und Desinfektionsbehandlung in den zwei oder mehr Reinigungsbehältern derart steuert, dass der Desinfektionsschritt in einem anderen Reinigungsbehälter als einem spezifischen Reinigungsbehälter, welcher für den Vorzugs-Reinigungsmodus eingerichtet wurde, gestartet wird, nachdem der Desinfektionsschritt in dem spezifischen Reinigungsbehälter, welcher für den Vorzugs-Reinigungsmodus eingerichtet wurde, abgeschlossen ist.

## Revendications

1. Système de nettoyage (10) pour endoscope, comprenant :
une pluralité de récipients de nettoyage (14a, b) dont chacun peut recevoir un endoscope (12) ;
au moins un réservoir à désinfectant (102) qui contient un désinfectant, un premier nombre desdits au moins un réservoir à désinfectant (102) étant inférieur à un second nombre de la pluralité de récipients de nettoyage (14a, b) ;
des moyens de fourniture de désinfectant pour fournir le désinfectant depuis ledit au moins un réservoir à désinfectant (102) vers la pluralité de récipients de nettoyage (14a, b) ;
des moyens de récupération de désinfectant (198a) pour récupérer le désinfectant depuis la pluralité de récipients de nettoyage (14a, b) vers ledit au moins un réservoir à désinfectant (102) ; et
une unité de commande (16) qui commande les moyens de fourniture de désinfectant et les moyens de récupération de désinfectant pour commander les étapes d'un traitement de nettoyage et de désinfection à exécuter dans la pluralité de récipients de nettoyage (14a, b) ;
**caractérisé en ce que** l'un desdits au moins un réservoir à désinfectant (102) a une capacité qui n'est pas inférieure à un volume de désinfectant requis pour exécuter une étape de désinfection dans l'un de la pluralité des récipients de nettoyage (14a, b), et ledit au moins un réservoir à désinfectant a une capacité totale qui est inférieure à un volume total du désinfectant requis pour exécuter l'étape de désinfection dans tous les récipients de nettoyage de la pluralité de récipients de nettoyage (14a, b).

2. Système de nettoyage pour endoscope selon la revendication 1, dans lequel le premier nombre desdits au moins un réservoir à désinfectant est égal à l'unité.

3. Système de nettoyage pour endoscope selon la revendication 1 ou 2, dans lequel le second nombre de la pluralité de récipients de nettoyage (14a, b) est égal à deux.

4. Système de nettoyage pour endoscope selon l'une quelconque des revendications 1 à 3, dans lequel l'unité de commande commande les étapes du traitement de nettoyage et de désinfection dans deux ou plusieurs récipients de nettoyage qui partagent un réservoir de désinfectant spécifique dudit au moins un réservoir à désinfectant de telle façon que lesdits deux ou plusieurs récipients de nettoyage ne vont pas exécuter l'étape de désinfection simultanément.

5. Système de nettoyage pour endoscope selon l'une quelconque des revendications 1 à 4, dans lequel, avant qu'un récipient de nettoyage dans les deux ou plusieurs récipients de nettoyage qui partagent le réservoir de désinfectant spécifique commence une étape de désinfection, l'unité de commande vérifie si le reste des deux ou plusieurs récipients de nettoyage se trouve dans l'étape de désinfection et, s'il est dans l'étape de désinfection, il amène le récipient de nettoyage à ne pas commencer l'étape de désinfection jusqu'à terminaison de l'étape de désinfection dans le reste des deux ou plusieurs récipients de nettoyage et, s'il n'est pas dans l'étape de désinfection, il amène le récipient de nettoyage à commencer l'étape de désinfection.

6. Système de nettoyage pour endoscope selon l'une quelconque des revendications 1 à 5, dans lequel l'unité de commande vérifie si les deux ou plusieurs récipients de nettoyage qui partagent le réservoir de désinfectant spécifique ont été réglés pour un mode de nettoyage préférentiel et elle commande les étapes du traitement de nettoyage de désinfection dans les deux ou plusieurs récipients de nettoyage de telle façon que l'étape de désinfection dans un autre récipient de nettoyage qu'un récipient de nettoyage spécifique qui a été réglé pour le mode de nettoyage préférentiel va être démarré après terminaison de l'étape de désinfection dans le récipient de nettoyage spécifique qui a été réglé pour le mode de nettoyage préférentiel.
